(19) Europäisches Patentamt
European Patent Office
Office européen des brevets

(11) Publication number: **0 079 792**
**B1**

(12) **EUROPEAN PATENT SPECIFICATION**

(45) Date of publication of patent specification: **08.07.87**

(21) Application number: **82306073.6**

(22) Date of filing: **15.11.82**

(51) Int. Cl.[4]: **C 12 N 9/80,** C 12 Q 1/58 // C12R1/01, C12R1/06, C12R1/13, C12R1/15, C12R1/37

# (54) Process for production of urease.

(30) Priority: **13.11.81 JP 182094/81**

(43) Date of publication of application:
**25.05.83 Bulletin 83/21**

(45) Publication of the grant of the patent:
**08.07.87 Bulletin 87/28**

(84) Designated Contracting States:
**DE FR GB IT**

(56) References cited:
**US-A-3 338 793**
**US-A-3 527 674**
**US-A-3 691 012**

**CHEMICAL ABSTRACTS, vol. 67, no. 7, August 14, 1967, page 2818, no. 29981q, Columbus, Ohio, US**

**CHEMICAL ABSTRACTS, vol. 50, no. 14, July 25, 1956, column 10187, reference a, Columbus, Ohio, US**

(73) Proprietor: **KYOWA HAKKO KOGYO CO., LTD**
**Ohtemachi Bldg. Ohtemachi 1-chome Chiyoda-ku**
**Tokyo (JP)**

(72) Inventor: **Tamura, Shigeaki**
**410-1, Nameri Nagaizumi-cho Sunto-gun**
**Shizuoka-ken (JP)**
Inventor: **Endo, Kenichi**
**310-2, Yasuhisa Mishima-shi**
**Shizuoka-ken (JP)**
Inventor: **Katsumata, Hideo**
**613-5, Kitahisahara Gotenba-shi**
**SHizuoka-ken (JP)**
Inventor: **Arai, Yuko**
**2016-40, Chabatake Susono-shi**
**Shizuoka-ken (JP)**

(74) Representative: **Lambert, Hugh Richmond et al**
**D. YOUNG & CO. 10 Staple Inn**
**London, WC1V 7RD (GB)**

**EP 0 079 792 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(56) References cited:
CHEMICAL ABSTRACTS, vol. 55, no. 10, May 15, 1961, column 9532, reference h, Columbus, Ohio, US

CHEMICAL ABSTRACTS, vol. 65, no. 4, August 15, 1966, column 5743, reference d, Columbus, Ohio, US

CHEMICAL ABSTRACTS, vol. 94, no. 21, May 25, 1981, page 298, no. 170070f, Columbus, Ohio, US

CHEMICAL ABSTRACTS, vol. 76, no. 19, May 8, 1972, page 237, no. 110215a, Columbus, Ohio, US

CHEMICAL ABSTRACTS, vol. 64, no. 4, February 14, 1966, column 5474, reference c, Columbus, Ohio, US

**Description**

The present invention relates to a process for production of urease.

Urease (E.C. 3, 5, 1, 5) is an enzyme which catalizes the decomposition reaction of urea into ammonia and carbon dioxide, and it is well known that urease is widespread in nature such as plants, animals and microorganisms. The production of urease from jack beans is already carried out on an industrial scale and therefore, urease is readily available. While many reports on studies of urease from microorganisms have been presented heretofore, the production of urea from microorganisms on an industrial scale is not yet practical because of poorer productivity than that of urease from jack beans.

On the other hand, urease is very useful in the determination of urea for diagnostic purpose. Urease from jack beans on the market, the Km value of which is $1{\sim}3\times10^{-3}$ using phosphate buffer (pH 7.5), suffers from a disadvantage of very limited determination ranges. It was reported in Journal of Bacteriology, *68*, 67~73 (1954) that urease, the Km value of which is larger than that of urease from jack beans, is prepared from a microorganism belonging to *Bacillus pasteurii*. However, because of poor sensitivity, this urease has difficulty in being used as an enzyme for diagnostic purpose.

Other items of prior art describing the preparation of urease from microorganisms include J. Gen. Microbiol. (1956), *14*, 478—484 which reports on the high urease activity of *Corynebacterium renale* (Chem. Abs. Vol. 50, No. 14, July 25, 1956 column 10187*a*); Lab. Delo 6, No. 6, 56—7 (1960) which reports on the activity of urease derived from *Proteus vulgaris* (Chem. Abs. Vol. 55, No. 10, May 15, 1961 column 9532*h*); Infect. Immun. 1981, 32(1), 32—37 which reports on the urease activity of a variety of microorganisms particularly of the genus *Proteus* (Chem. Abs. Vol. 94, No. 21, May 25, 1981, 170070f); and Arch. Mikrobiol. 1972, 81(2), 178—96 (Eng) which reports on the urease activity of selected aerobic bacteria (Chem. Abs. Vol. 76, No. 19, May 8, 1972, 110215a).

Enzymatic assays using urease are reported in Anal. Biochem. 16(1), 132—8 (1966) Eng. (Chem. Abs. Vol. 65, No. 4, Aug 15, 1966, 5743) and US—A—3,527,674.

Microorganisms having urease activity are also reported in US—A—3,338,795 and 3,691,012.

In accordance with the present invention other microorganism urease sources have now been found which produce in good yield ureases having a high Km value (in the range $1{\sim}5\times10^{-2}$ as determined using urea as a substrate in a phosphate buffer at pH 7.5 and at a temperature of 37°C) and which can be used in urea assays of high sensitivity.

In accordance with the present invention, such high Km value ureases are obtained by culturing a microorganism of the species *Corynebacterium lilium*, or of the genus *Brevibacterium, Arthrobacter, Microbacterium* or *Bordetella* which is capable of producing the enzyme, in a nutrient medium until the urease is accumulated in the culture liquor and subsequently recovering the accumulated urease.

Any microorganism of the species *Corynebacterium lilium* or of the genus *Brevibacterium, Arthrobacter, Microbacterium* or *Bordetella* capable of producing urease having Km value in the range $1{\sim}5\times10^{-2}$ can be used. Preferred microorganisms are:

(1) *Corynebacterium lilium* ATCC 21644
(2) *Brevibacterium ammoniagenes* ATCC 6872
(3) *Arthrobacter parafineus* ATCC 15591
(4) *Microbacterium ammoniaphilum* ATCC 15354 and
(5) *Bordetella bronchiseptica* ATCC 4617

Either a synthetic or natural medium may be used for culturing of the microorganism as long as it contains an appropriate carbon source, nitrogen source, inorganic materials and other nutrients which are assimilable by the particular strain utilized.

As a carbon source, various carbohydrates such as glucose, fructose, sucrose, dextrin, molasses, starch, etc., alcohols such as glycerol, sorbitol, manitol, etc., organic acids such as uric acid, acetic acid, pyruvic acid, etc., amino acids, hydrocarbons and the like may be used.

As nitrogen sources, ammonia, inorganic and organic ammonium salts such as ammonium chloride, ammonium carbonate, ammonium phosphate, ammonium sulfate, ammonium nitrate, ammonium acetate etc., urea, amino acids such as glutamic acid and other nitrogen-containing compounds as well as nitrogenous organic materials such as peptone, NZ-amine, meat extract, yeast extract, corn steep liquor, soybean powder, defatted soybean, dry yeast, casamino acid, soluble vegetable protein and the like may be used.

As inorganic materials, potassium dihydrogen phosphate, dipotassium hydrogen phosphate, magnesium sulfate, ferrous sulfate, manganese sulfate, zinc sulfate, sodium chloride, potassium chloride, calcium carbonate, etc. may be used.

Culturing is generally carried out at a temperature of 20—40°C, preferably 25—35°C and at a pH of 6—8. Culturing is continued until the enzyme is formed and detected in the culture liquor, generally for 1—4 days. Under these conditions, a considerable amount of the enzyme is formed in the culture liquor and/or within the microbial cells.

After completion of the culturing, the microbial cells are disrupted by appropriate means such as ultrasonic disintegration, grinding, etc. to obtain a cell extract and the extract is subjected to centrifugation to obtain a supernatant. Then the supernatant is subjected to conventional purification methods such as

salting-out, dialysis, chromatography using ion exchange cellulose, Sephadex, and the like to obtain the purified enzyme.

In order to calculate the enzymatic activity of urease, ultraviolet absorption method, which is readily used as a diagnostics, is chosen among various methods. The enzymatic activities of urease is calculated by decomposing urea as a substrate in the presence of urease which catalyzes a reaction in which urea is hydrolyzed into ammonia, reacting said ammonia with α-ketoglutaric acid in the presence of glutamate dehydrogenase (hereinafter referred to as "GLDH") and reduced nicotinamide adenine dinucleotide (phosphate) (hereinafter referred to as "NAD(P)H") as a hydrogen donor and measuring the decreasing rate of ultraviolet absorbancy due to decrease in NAD(P)H.

(A) Reagents

| | | | |
|---|---|---|---|
| 1) | 0.1 M | Phosphate buffer (pH 7.5) | 2.60 ml |
| 2) | 15 mg/ml | α-ketoglutaric acid | 0.10 ml |
| 3) | 20 mg/ml | urea | 0.15 ml |
| 4) | 2.5 mg/ml | NAD(P)H | 0.10 ml |
| 5) | 1500 U/ml | GLDH | 0.02 ml |

(B) Operations

The above reagents are mixed with each other and incubated at 37°C for 5 minutes. To the mixture is added 0.01 ml of an enzyme solution, the enzymatic activity of which is to be determined. The absorbancy at $OD_{340nm}(=E_1)$ after one minute and that at $OD_{340nm}(=E_2)$ after two minutes are recorded at 37°C on a spectrophotometer, respectively.

The enzymatic activity (A) can be calculated from:

$$A(U/ml)=(E_2-E_1)\times\frac{1}{12.44}\times\frac{2.98}{0.01}$$

One unit of urease is defined as the amount of the enzyme which forms one μmole of ammonia for one minute.

A urease obtained as above can be used in a method and composition for the determination of urea in a sample. In this method the urea in the sample is reacted with the enzyme and the amount of ammonia formed in the enzymatic reaction is determined. The formed ammonia may be determined by a known method. For example, a preferred method is as follows.

Ammonia is reacted with α-ketoglutaric acid in the presence of GLDH and NAD(P)H to form glutamic acid and nicotinamide adenine dinucleotide(phosphate) [hereinafter referred to as NAD(P)].

The amount of NAD(P)H is determined by measuring the absorbancy of the reaction solution at an ultraviolet ray region, for example 340 nm. The concentration of urea in the sample is calculated by measuring the decreasing rate of NAD(P)H.

The present method is schematically shown below.

$$\text{Urea}+H_2O\xrightarrow{\text{Urease}}2NH_3+CO_2$$

$$NH_3+\alpha\text{-Ketoglutaric acid}\xrightarrow{\text{GLDH}}\text{Glutamic acid}+H_2O$$

NAD(P)H → NADP

The ingredients of the enzymatic reaction solution are used in the following concentrations.

Urease: 0.1~10 U/ml, preferably 0.3~1U/ml
GLDH: 1~100 U/ml, preferably 7~30 U/ml
NAD(P)H: 0.01 mM~1 mM, preferably 0.1~0.25 mM
α-ketoglutaric acid: 1~50 mM, preferably 5~15 mM

The determination may be performed by conducting the individual reactions stepwise and may preferably be performed in one step by adding the ingredients necessary for the determination.

However, if a sample contains ammonia, GLDH and α-ketoglutaric acid is first added to the sample to decompose the ammonia and then urease is added to the reaction mixture.

The enzymatic reaction is carried out at 20~75°C, preferably 30~50°C in an appropriate buffer solution.

As a buffer solution, borate buffer, phosphate buffer, tris-hydrochloride buffer, tris-maleate-

hydrooxide buffer, citrate-disodium phosphate buffer, succinate-borate buffer, phosphate-borate buffer and the like are used in a concentration of 0.005—0.5 mol/l.

The test composition comprises a system (A) for decomposing urea and a system (B) for determining ammonia.

The system (A) comprises urease and system (B) comprises GLDH, α-ketoglutaric acid and NAD(P)H. The composition may be used in various forms. For example, the ingredients may be mixed in liquid form or powder form. The powdered formulation may be readily reconstituted for later use simply by the addition of buffer solution.

The composition may contain stabilizer of enzyme, buffer, surfactant, etc.

Certain specific embodiments of the invention are illustrated by the following representative examples.

Example 1

In this example, 30 ml of the first seed medium (pH 7.2) comprising 1 g/dl peptone, 1 g/dl meat extract and 0.3 g/dl sodium chloride is put into a 250 ml—Erlenmeyer flask and sterilized at 120°C for 15 minutes (the media as used hereinafter are sterilized in the same manner). *Corynebacterium lilium* ATCC 21644 is used as a seed strain. One loopful of said strain is inoculated into the seed medium and cultured with shaking at 28°C for 24 hours. Then, 30 ml of the seed culture liquor is put into a 2 l-Erlenmeyer flask containing 300 ml of a culture medium comprising the same components as the first seed medium and cultured with shaking at 28°C for 24 hours. Thereafter, 1 l of the second seed culture liquor thus obtained is poured into a 30 l-jar fermenter containing 15 l of a fermentation medium (pH 7.2) comprising 10 g/dl glucose, 20 g/dl CSL, 1 g/dl ammonium sulfate, 0.5 g/dl potassium hydrogen phosphate, 0.5 g/dl potassium chloride and 0.5 g/dl magnesium sulfate. Main fermentation is carried out with aeration-stirring at an aeration rate of 15 l/minute, a stirring speed of 30 r.p.m. and 28°C for 48 hours. The enzymatic activity of urease contained in the resulting culture liquor is 2.1 U/ml.

After the completion of culturing, 16 l of the culture liquor is centrifuged to collect microbial cells. The microbial cells are suspended and disrupted in one l of 0.03M phosphate buffer (pH 7.0). The resulting disrupted cell suspension is centrifuged to obtain a supernatant containing the crude enzyme. To the supernatant is added ammonium sulfate to 70% saturation to form a precipitate. The precipitate is recovered by centrifugation and dissolved in 50 ml of 0.03M phosphate buffer (pH 7.0). The resulting solution is dialyzed against 5 l of the same phosphate buffer using a cellophane tube as a dialysis membrane at 4°C overnight. The thus obtained concentrated enzyme solution is passed through a column of 200 ml of DEAE-cellulose (product of Pharmatica Fine Chemicals, Sweden) to effect adsorption of urease. The active fractions of urease are eluted with 0.4M NaCl and the enzymatic activity thereof is 40 U/mg protein. The eluent is added to 0.1 mg/U albumin or lactose as a stabilizer, concentrated and freeze-dried, whereby about 400 mg of an enzyme preparate is obtained. The properties of the enzyme preparate are described below.

| | |
|---|---|
| (1) Action: | This enzyme catalyzes the reaction in which one mole of urea is hydrolyzed consuming one mole of water into two moles of ammonia and one mole of carbon dioxide. |
| (2) Optimum pH: | 7.5—8.0 |
| (3) pH Stability: | The present enzyme is stable at a pH from 7 to 10 with treatment at 37°C for 30 minutes. |
| (4) Optimum temperature: | 45—50°C |
| (5) Heat stability: | This enzyme is stable upto 50°C with treatment at pH 7 for 30 minutes. |
| (6) Substrate specificity: | The relative activity on several substrate is as follows: |

| Substrate | Relative activity(%) |
|---|---|
| Urea | 100 |
| Burette | 13 |
| Allantoin | 11 |

(7) Inhibition

| Inhibiting substances | Concentration (M) | Percent inhibition (%) |
|---|---|---|
| — | | 100 |
| $AgNO_3$ | $1\times10^{-5}$ | 11 |
| $CuSO_4$ | $1\times10^{-5}$ | 10 |
| Guanidine hydrochloride | $4\times10^{-3}$ | 31 |

| | |
|---|---|
| (8) Km value: | The Km value of this enzyme is determined using phosphate buffer (pH 7.5), to be $2\times10^{-2}$ which is about 10 times as large as that of urease from jack beans. |
| (9) | It is hardly possible to crystallize this enzyme. |
| (10) Molecular weight: | The molecular weight of this enzyme is calculated to be about 440,000 by Sephadex G-200 gel-filtration method. |
| (11) Elementary analysis: | It is not measured because this enzyme is not crystallized. |

Example 2

In this example, each of the strains identified in the following Table 1 is cultured in the same manner as described in Example 1 to obtain microbial cells. From the microbial cells, a crude enzymatic solution is prepared to calculate the enzymatic activity and to determine the Km value. Table 1 shows that the Km values of the strains are five to twenty times as large as that of urease from jack beans.

TABLE 1

| Strain | Enzymatic activity (U/ml) | Km value ($\times10^{-2}$) |
|---|---|---|
| *Brevibacterium ammoniagenes* ATCC 6872 | 0.6 | 1.7 |
| *Arthrobacter parafineus* ATCC 15591 | 0.5 | 2.0 |
| *Microbacterium ammoniaphilum* ATCC 15354 | 3.5 | 2.5 |
| *Bordetella bronchiseptica* ATCC 4617 | 0.4 | 4.4 |

Example 3

In this example, the determination of urea by using a urease according to the present invention is carried out.

3 ml of 0.1M Phosphate buffer (pH 8.0) containing 0.5 U/ml urease of Example 1, 10 U/ml GLDH, 0.2 mM NADPH and 10 mM α-ketoglutaric acid is incubated at 37°C for 5 minutes. To the reagent solution is added 50 µl of sample and the mixture is incubated at 37°C. Two minutes ($T_1$) and four minutes ($T_2$) after the addition, the absorbancies of the reaction solution at 340 nm are measured.

The same procedure as described above are repeated for the standard sample (50 mg/dl) to obtain the absorbancies ($S_1$, 2 minutes after the addition and $S_2$, 4 minutes after the addition). The urea concentration (A) of the sample is calculated according to the following equation.

$$A=\frac{T_1-T_2}{S_1-S_2}\times50$$

**Claims**

1. A process for producing urease which comprises culturing a microorganism capable of producing urease in a nutrient medium, allowing the urease to accumulate in said medium and subsequently recovering the accumulated urease from the medium, characterised in that the urease has a Km value (determined using urea as a substrate in a phosphate buffer at pH 7.5 and at a temperature of 37°C) in the range $1\sim5\times10^{-2}$ and is obtained using a microorganism of the species *Corynebacterium lilium*, or belonging to the genus *Brevibacterium, Arthrobacter, Microbacterium* or *Bordetella*.

2. A process according to claim 1, characterised in that the microorganism used is of the species *Brevibacterium ammoniagenes, Arthrobacter parafineus, Microbacterium ammoniaphilum* or *Bordetella bronchiseptica*.

3. A process according to claim 1, characterised in that the microorganism is *Corynebacterium lilium* ATCC 21644, *Brevibacterium ammoniagenes* ATCC 6872, *Arthrobacter parafineus* ATCC 15591, *Microbacterium ammoniaphilum* ATCC 15354 or *Bordetella bronchiseptica* ATCC 4617.

4. A process according to claim 1, 2 or 3, characterised in that the microorganism is cultured in said medium at 20 to 40°C for 1 to 4 days at a pH of 6—8.

**Patentansprüche**

1. Verfahren zur Herstellung von Urease durch Züchtung eines zur Herstellung von Urease befähigten Mikroorganismus in einem Nährmedium, Ansammelnlassen der Urease in dem Medium und anschließend Gewinnung der angesammelten Urease aus dem Medium, dadurch gekennzeichnet, daß die Urease einen Km-Wert (bestimmt unter Verwendung von Harnstoff als Substrat in einem Phosphat-Puffer bei pH 7,5 und einer Temperatur von 37°C) im Bereich von 1 bis $5\times10^{-2}$ aufweist und unter Verwendung eines

Mikroorganismus der Spezies *Corynebacterium lilium*, oder eines zur Gattung *Brevibacterium, Arthrobacter, Microbacterium* oder *Bordetella* gehörenden Mikroorganismus erhalten wird.

2. Verfahren nach Anspruch 1, dadurch gekennzeichnet, daß der verwendete Mikroorganismus zur Spezies *Brevibacterium ammoniagenes, Arthrobacter parafineus, Microbacterium Ammoniaphilum* oder *Bordetella bronchiseptica* gehört.

3. Verfahren nach Anspruch 1, dadurch gekennzeichnet, daß der Mikroorganismus *Corynebacterium lilium* ATCC 21644, *Brevibacterium ammoniagenes* ATCC 6872, *Arthrobacter parafineus* ATCC 15591, *Microbacterium ammoniaphilum* ATCC 15354 oder *Bordetella bronchiseptica* ATCC 4617 ist.

4. Verfahren nach Anspruch 1, 2 oder 3, dadurch gekennzeichnet, daß der Mikroorganismus in dem Medium bei 20 bis 40°C 1 bis 4 Tage bei einem pH von 6 bis 8 gezüchtet wird.

**Revendications**

1. Procédé pour la production d'uréase qui comprend le fait de cultiver un microorganisme capable de produire de l'uréase dans un milieu nutritif, de laisser l'uréase s'accumuler dans ledit milieu et ensuite de récupérer de ce milieu l'uréase accumulée, caractérisé en ce que l'uréase a une valeur Km (déterminée en utilisant l'urée à titre de substrat dans un tampon phosphate à pH 7,5 et à une température de 37°C) dans l'intervalle $1\sim5\times10^{-2}$ et en ce qu'elle est obtenue en utilisant un microorganisme de l'espèce *Corynebacterium lilium*, ou appartenant au genre *Brevibacterium, Arthrobacter, Microbacterium* ou *Bordetella*.

2. Procédé selon la revendication 1, caractérisé en ce que le microorganisme utilisé est de l'espèce *Brevibacterium ammoniagenes, Arthrobacter parafineus, Microbacterium ammoniaphilum* ou *Bordetella bronchiseptica*.

3. Procédé selon la revendication 1, caractérisé en ce que le microorganisme est *Corynebacterium lilium* ATCC 21644, *Brevibacterium ammoniagenes* ATCC 6872, *Arthrobacter parafineus* ATCC 15591, *Microbacterium ammoniaphilum* ATCC 15354 ou *Bordetella bronchiseptica* ATCC 4617.

4. Procédé selon l'une quelconque des revendications 1, 2 ou 3, caractérisé en ce que le microorganisme est cultivé dans ledit milieu entre 20 et 40°C, pendant 1 à 4 jours et à pH 6—8.